(19) Europäisches Patentamt
European Patent Office
Office européen des brevets

(11) **EP 1 854 813 A1**

(12) **EUROPEAN PATENT APPLICATION**
published in accordance with Art. 158(3) EPC

(43) Date of publication:
**14.11.2007 Bulletin 2007/46**

(51) Int Cl.:
***C08B 37/00*** (2006.01)

(21) Application number: **06728569.2**

(22) Date of filing: **01.03.2006**

(86) International application number:
**PCT/JP2006/303834**

(87) International publication number:
**WO 2006/093175 (08.09.2006 Gazette 2006/36)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.03.2005 JP 2005055408**

(71) Applicant: **Ube Industries, Ltd.**
**Ube-Shi,**
**Yamaguchi 755-0052 (JP)**

(72) Inventors:
• **HATANO, Koji**
**978-5, Kogushi, Ooaza, Ube-shi, Yamaguch (JP)**
• **NAKAMOTO, Yasushi**
**978-5, Kogushi, Ooaza, Ube-shi, Yamaguch (JP)**
• **KANETSUKI, Yuu c/o Ube Industries, Ltd. Ube**
**Ube-shi, Yamaguchi, 75586 33 (JP)**

(74) Representative: **Dossmann, Gérard**
**Bureau D.A. Casalonga-Josse**
**Bayerstrasse 71/73**
**80335 München (DE)**

(54) **HIGH-MOLECULAR FUCOIDAN, METHOD OF PRODUCING THE SAME AND COSMETIC COMPOSITION**

(57) The present invention provides a method for producing high molecular-weight fucoidan, which is expected to be promising as a cosmetic composition or an agent in the field of dermatology, and a high molecular-weight fucoidan-containing composition.

A component containing fucoidan having a weight average molecular weight of 1,000,000 to 2,000,000 obtained through the steps of performing hot water extraction of a mozuku alga body of the genus Cladosiphon at pH 6.0 or higher, which ranges from neutral to alkaline, removing low-molecular weight compound through ultrafiltration, and adjusting a final pH to 6.5 or higher is effective as a cosmetic composition or an agent in the field dermatology, and is also excellent in storage stability.

EP 1 854 813 A1

**Description**

BACKGROUND OF THE INVENTION

Field of the Invention

The present invention relates to a method for producing high molecular-weight fucoidan having a weight average molecular weight of 1,000,000 to 2,000,000 which is obtained from seaweed, to high molecular-weight fucoidan and a fucoidan-containing composition containing the high molecular-weight fucoidan and, more particularly, to a composition for the field of dermatology (a cosmetic composition, a skin cell activator, and an anti-allergic agent).

Description of the Related Art

Fucoidan is a viscous component contained in brown algae such as mozuku and tangle, which is a kind of an acidic polysaccharide containing sulfated fucose as the main constituent saccharide. Fucoidan has various pharmacological actions such as anti-tumor, anti-gastric ulcer, antiviral, anti-inflammation, anti-blood coagulation and immunologic enhancement actions. In addition, fucoidan has a moisture retaining function, a skin cell activating action, an anti-allergic action, an agglutinative action, a thickening action and a surface-active action. For this reason, fucoidan is a promising substance in the fields of cosmetic compositions and dermatological agents.
Many technologies have been disclosed in regard to methods for producing fucoidan or fucoidan-containing components from brown algae such as mozuku, tangle, brown seaweed and fucus.
The "fucoidan-containing component" in the present specification means a solid or liquid substance obtained by using an optional method for producing fucoidan, comprising extracting fucoidan from a raw alga body.
In a common method for producing fucoidan, fucoidan is extracted under acidic conditions (Patent Documents 1 to 7).
However, fucoidan structure has monosaccharides bonded by an acetal bond, and therefore it creates a problem that the molecular weight of fucoidan is decreased by heating under acidic conditions or by long-term storage.
Fucoidans having various molecular weights are known.
For example, in Patent Document 6, fucoidan having a weight average molecular weight of 500, 000 obtained from cultured Ito-mozuku seaweed (Nemacystus decipiens) is described.
Moreover, in Patent Document 7, fucoidan having a weight average molecular weight of 500,000 to 600,000 obtained from cultured Okinawa-mozuku seaweed (Cladosiphon okamuranus) is described.
In Patent Document 3, fucoidan having a weight average molecular weight of 10,000 to 500,000 obtained from Okinawa-mozuku seaweed by hot water extraction at pH 2 to 8 under pressure in an autoclave is described.
In Patent Document 4, fucoidan having a weight average molecular weight of 180,000 obtained from Okinawa-mozuku seaweed by extraction with organic acid is described.
In Patent Document 8, fucoidan having a weight average molecular weight of about 100,000 obtained from mozuku seaweed by hot water extraction is described.
Furthermore, in. Patent Document 9, fucoidan having a weight-average molecular weight of 50,000 to 500,000 obtained by subjecting Hoso-mozuku seaweed (Nemacystus decipiens) to hot water extraction under neutral conditions is described.
However, a method for producing fucoidan having a weight average molecular weight of more than 1,000,000 obtained from a natural product as a source has never been proposed.
Fucoidan having a weight average molecular weight of 909, 000 is described in Non-Patent Document 2. However, pullulan is used as a reference standard for the measurement of the molecular weight of the fucoidan. Pullulan is a straight chain polysaccharide, whereas, fucoidan is a polysaccharide having many branches. Hence, there is a large difference between the two substances in terms of the relationships between their respective molecular weight and molecular size. Therefore, the molecular weight of a branched polysaccharide such as fucoidan measured using pullulan as a reference standard is drastically larger than that measured using a multi-angle light scattering detector. The present inventors actually measured the molecular weight of fucoidan using pullulan as a reference standard. As a result, the weight average molecular weight measured using a multi-angle light scattering detector was 1, 845, 000 (see, Example 3), whereas the molecular-average weight as measured using pullulan as a reference standard was 17,400,000 (see, Reference Example 1).
Cosmetic compositions are composed of various components. Thickening agents in these components maintain the viscosity of chemicals in the cosmetic compositions. Not only do thickening agents contribute to stability of the compositions together with emulsifiers, but also have a large effect on tactile sensations (such as smoothness, moist feeling and non-stickiness) of cosmetic compositions (Non-Patent Document 1).
Fucoidan separated and purified from tangle, arame (Eisenia bicyclis) and brown seaweed is gaining attention for its viscosity and being applied to cosmetic compositions (Patent Document 11). Patent Document 11 describes that, when a cosmetic composition containing fucoidan is applied to the skin, smoothness and excellent tactile sensation are observed, which maintain the skin in a moist and smooth state.

In Patent Document 12, fucoidans from the genera Analipus, Nemacystus, Ecklonia, Lessonia, Macrosystis, Fucus, Ascophyllum and Durvillea of brown algae have a biologic hyaluronan synthesis action, a hyaluronidase inhibitory action and a histamine release inhibitory action.

However, it is not proposed that fucoidan derived from the genus Cladosiphon be used as a cosmetic composition or an agent in the field of dermatology.

As described above, there is a high expectation for fucoidan as a cosmetic composition and as an agent in the field of dermatology because it has an anti-allergic action. However, there has never been a case in which the relationship between functions of fucoidan such as anti-allergic action, viscosity and tactile sensation, which are important in these fields, and the weight average molecular weight of fucoidan is studied.

Patent Document 1: Japanese Unexamined Patent Publication (Kokai) No. 10-191940.
Patent Document 2: Japanese Unexamined Patent Publication (Kokai) No. 11-35591.
Patent Document 3: Japanese Unexamined Patent Publication (Kokai) No. 2000-239301.
Patent Document 4: Japanese Unexamined Patent Publication (Kokai) No. 2000-239302.
Patent Document 5: Japanese Unexamined Patent Publication (Kokai) No. 2000-351801.
Patent Document 6: Japanese Unexamined Patent Publication (Kokai) No. H10-195106.
Patent Document 7: Japanese Unexamined Patent Publication (Kokai) No. H10-237103.
Patent Document 8: Japanese Unexamined Patent Publication (Kokai) No. H07-138166.
Patent Document 9: Japanese Unexamined Patent Publication (Kokai) No. H10-70970.
Patent Document 10: Japanese Examined Patent Publication (Kokoku) No. H06-11763.
Patent Document 11: Japanese Examined Patent Publication (Kokoku) No. H07-14850.
Patent Document 12: Japanese Unexamined Patent Publication (Kokai) No. H11-21247.
Patent Document 13: Japanese Unexamined Patent Publication (Kokai) No. 2001-151788.
Non-patent Document 1: "New Cosmetology" edited by Takeo Mitsui, the fourth impression of the first edition, published by Nanzando, 1999, pp.5-7, pp.147-148
Non-patent Document 2: "Riken Mekabufukoidan", [online], [searched on 22 February, 2006], Internet<http://www.rikenvitamin.jp/prouse/health/meka bufukoidan.html>

Accordingly, an object of the present invention is to provide a method for producing high molecular-weight fucoidan, which is expected to be promising as a cosmetic composition and an agent in the field of dermatology, and a cosmetic composition containing the high molecular-weight fucoidan.

SUMMARY OF THE INVENTION

The present inventors have intensively studied so as to solve the above problem and thus obtained a component containing fucoidan having a weight average molecular weight of 1,000,000 to 2,000,000 through the steps of performing hot water extraction of a mozuku alga body of the genus Cladosiphon at pH 6.0 or higher, which ranges from neutral to alkaline, removing low-molecular weight compounds through ultrafiltration, and adjusting the final pH to 6.5 or higher. Furthermore, it has been found that fucoidan thus obtained exists stably for a long period of time. Thus, the present invention has been completed.

That is, a first invention is a method for producing fucoidan comprising the steps of performing hot water extraction of a mozuku alga body of the genus Cladosiphon at pH 6.0 or higher, removing low-molecular weight compounds while maintaining the hot water at pH 6.5 or higher, and adjusting the final pH to 6.5 or higher to obtain fucoidan having a weight average molecular weight of 1,000,000 to 2,000,000.

A second invention is fucoidan obtained by the method of the first invention.

A third invention is fucoidan having a weight average molecular weight of 1,000,000 to 2,000,000.

A fourth invention is fucoidan according to the second or third invention, wherein an aqueous solution thereof having a dried residue weighing 1.0 to 2.0% by weight has a viscosity of from 600 to 1,500 mPa·s as measured at 20 rpm and 25°C using a C-type viscometer.

A fifth invention is a fucoidan-containing composition, comprising fucoidan according to any one of the second to fourth inventions.

A sixth invention is a fucoidan-containing composition according to the fifth invention, wherein the fucoidan-containing composition is a cosmetic composition, a skin cell activator or an anti-allergic agent.

According to the method of the present invention, fucoidan having a weight average molecular weight of 1,000,000 to 2,000,000 is obtained. This fucoidan has a high molecular-weight which has never been obtained before by prior art methods. Such fucoidan is not only excellent in moisture retention, and effective as a component of fucoidan-containing compositions for cosmetic compositions and agents in the fields of dermatology, but also excellent in storage stability.

DESCRIPTION OF THE PREFFERED EMBODIMENTS

Mozuku seaweed used in the present invention as a source of fucoidan is preferably a mozuku seaweed of the genus Cladosiphon because of its high content of high-molecular weight fucoidan. Moreover, the mozuku seaweed may be used in a raw state, a dried state, or dehydrated with alcohol.

The high-molecular fucoidan having a weight average molecular weight of 1, 000, 000 to 2, 000, 000 obtained by the method of the present invention can be preferably mixed as a component of compositions such as cosmetic compositions (skin lotion, hair treatment agent, etc.) and agents in the field of dermatology(skin cell activator or anti-allergic agent).

Thereafter, the method for producing high molecular-weight fucoidan of the present invention will now be described with respect to each step in this method.

<Step of Hot Water Extraction>

The extraction operation is performed after mozuku seaweed is suspended in tap water or purified water to obtain a suspension. The amount of the water used is not specifically limited as long as fucoidan can be efficiently extracted. For example, in case of using dried mozuku seaweed, it is preferred to use water in the amount of 50 to 300 g per g of dried mozuku seaweed.

When extraction is initiated, a hydroxide of an alkaline metal salt, such as sodium hydroxide or potassium hydroxide, or a hydroxide of an alkaline earth metal element, such as calcium hydroxide or barium hydroxide is dissolved in the suspension. Among these hydroxides, potassium hydroxide, which can also be used as a food additive, is preferable. The concentration of the aqueous alkaline solution is preferably from 0.005 to 0.1 mol/L (liter).

When fucoidan is extracted, an appropriate amount of hydrogen peroxide water having a concentration of 30% by weight (for example, 0- to 2-fold amount based on the weight of dried mozuku seaweed in case of using dried mozuku seaweed) may be used so as to decolorize or deodorize the fucoidan-containing solution.

The extraction temperature of hot water extraction is preferably from 70 to 100°C and the extraction time is preferably from 0.5 to 2 hours. This is because the molecular weight of the fucoidan is decreased if the extraction temperature is too high or the extraction time is too long. The extraction water is maintained at pH 6.0 or higher.

After extraction, the extraction residue in the suspension is removed using an apparatus capable of efficiently filtrating viscous substances, such as a centrifuge separator or a filter press. During removal, a filter aid such as diatomaceous earth may be added. Activated carbon may be added before the filtration so as to decolorize more completely.

<Step of Removing Low-Molecular Weight Compound>

After removing the extraction residue, the resulting transparent fucoidan-containing solution is subjected to a method such as ultrafiltration or dialysis in order to remove low-molecular weight compounds. Ultrafiltration is preferable in view of time efficiency. It is preferable to use an ultrafiltration membrane having a fractional molecular weight of 6,000 to 10,000. Since operation under acidic conditions tends to cause the molecular weight to decrease, it is necessary that the fucoidan-containing liquid is maintained at pH 6.5 or higher, preferably 6.5 to 7.5, until the the removal operation of the low-molecular weight compounds is completed.

<Step of pH Adjustment>

After ultrafiltration, the fucoidan-containing solution may be neutralized with a mineral acid such as hydrochloric acid or sulfuric acid. However,it is necessary to maintain the fucoidan-containing solution at pH 6.5 or higher, and preferably 6.5 to 7.5, in view of storage stability and the use of the solution as a cosmetic composition. This is because the fucoidan-containing solution is preferably weakly acidic to neutral in view of dermal irritation.

The resulting fucoidan-containing liquid, which is an aqueous transparent solution, may be used as it is as a material for cosmetic compositions, or powderized by the method of spray drying or freeze drying. Alternatively, alcohol is added to the solution and the resulting precipitate is collected by filtration, which is then dried by a conventional method. However, in case of drying the filtrated precipitate with heating, the precipitate is preferably heated at 70°C or lower so as to avoid a decrease in the molecular weight.

EXAMPLES

<Example 1>

Ethanol was added to a raw alga body of Cladosiphon novae-caledoniae so as to obtain a concentration of 50% by weight. After dipping the raw mozuku seaweed for 30 minutes for discoloration, as much ethanol was removed as possible by centrifugal filtration to prepare processed mozuku seaweed.

150 g of the processed mozuku seaweed was put in 1, 350 ml of water and then 13.2 g of hydrogen peroxide water having a concentration of 30% by weight and 0.84 g of potassium hydroxide were added, followed by stirring at 90 to 95°C for one hour. After cooling to 50°C, the residue was removed by filtration.

Since the resulting filtrate was pH 6.3, 1 mol/L (liter) of an aqueous potassium hydroxide solution was used to adjust the pH to 7.2.

Then, the filtrate was concentrated to about 1/5 of the original volume using an ultrafiltration membrane having a fractional molecular weight of 10,000.

The resulting aqueous solution was adjusted to pH 6.5 using 1 mol/L (liter) of dilute sulfuric acid.

Furthermore, the solution was treated with catalase to decompose peroxide so as to obtain an aqueous fucoidan solution. The weight of the dried residue of the above-mentioned aqueous fucoidan solution (at 70°C for 8 hours under normal pressure) was 1.2% by weight based on the original aqueous solution.

Also, fucoidan in the aqueous fucoidan solution was ethanol-precipitated (see, Example 2), and the weight average molecular weight was measured by a multi-angle light scattering detector. As a result, it was 1,845,000 (see, Example 3). Hereinafter, this aqueous fucoidan solution is referred to as an "aqueous high molecular-weight fucoidan solution (Example 1)".

<Comparative Example 1>

Ethanol was added to a raw alga body of Cladosiphon novae-caledoniae so as to obtain a concentration of 50% by weight. After dipping the raw mozuku seaweed for 30 minutes for discoloration, as much ethanol was removed as possible by centrifugal filtration to prepare processed mozuku seaweed.

150 g of the processed mozuku seaweed was put in 1, 350 ml of water and 13.2 g of hydrogen peroxide water having a concentration of 30% by weight was added. Then 1 mol/L (liter) of dilute sulfuric acid was used to adjust the pH to 3, followed by stirring at 90 to 95°C for one hour. After cooling to 50°C, the residue was removed by filtration.

Then, the filtrate was concentrated to about 1/5 of the original volume using an ultrafiltration membrane having a fractional molecular weight of 10,000.

The resulting aqueous solution was adjusted to pH 6.5 using 1 mol/L (liter) of potassium hydroxide.

Furthermore, the solution was treated with catalase to decompose peroxide so as to obtain an aqueous fucoidan solution. Hereinafter, this aqueous fucoidan solution is referred to as an "aqueous acid-extracted fucoidan solution".

<Example 2> Preparation of Ethanol Precipitate

Preparation of Ethanol Precipitate of High Molecular-Weight Fucoidan

100 g of the aqueous high molecular-weight fucoidan solution obtained in Example 1 was concentrated to about 1/2 of the original volume by removing the water under reduced pressure. A 4-fold volume of ethanol was added to the resulting concentrated solution, thereby precipitating fucoidan. The precipitate was separated by centrifugation (at 3, 000 rpm for 5 minutes at 4°C). Then, the resulting residue was washed twice with ethanol and dried at 50°C under a reduced pressure (0.001 MPa) to prepare an ethanol precipitate (0.876 g) of high molecular-weight fucoidan.

Hereinafter, this precipitate is referred to as "high molecular-weight fucoidan".

<Comparative example 2> Preparation of Aqueous Hydrolyzed Fucoidan Solution and Ethanol Precipitate of the Same

100 g of the aqueous high molecular-weight fucoidan solution obtained in Example 1 was adjusted to pH 5 using 2N sulfuric acid, followed by heat treatment at different temperatures for different lengths of time (at 70°C for one hour, at 70°C for 2 hours, at 90°C for one hour) to obtain aqueous hydrolyzed fucoidan solutions.

Hereinafter, these aqueous hydrolyzed fucoidan solutions are respectively referred to as " aqueous hydrolyzed fucoidan solution (at 70°C for one hour)"," aqueous hydrolyzed fucoidan solution (at 70°C for 2 hours) " and "aqueous hydrolyzed fucoidan solution (at 90°C for one hour)".

100 ml of each of these aqueous hydrolyzed fucoidan solutions was ethanol-precipitated in the same manner as the previous examples so as to obtain an ethanol precipitate of hydrolyzed fucoidan.

Hereinafter, these ethanol precipitates of hydrolyzed fucoidan are respectively referred to as "hydrolyzed fucoidan (at 70°C for one hour)", "hydrolyzed fucoidan (at 70°C, for 2 hours)" and "hydrolyzed fucoidan (at 90°C for one hour)".

<Example 3> Measurement of Weight Average Molecular Weight

Each of the ethanol precipitates of the high molecular-weight fucoidan obtained in Example 2 and the hydrolyzed fucoidan obtained by Comparative Example 2 were dissolved in an aqueous sodium chloride solution having a concentration of 0.1 mol/L (liter) to obtain a solution having a concentration of 0.05% by weight. Then these solutions were subjected to high-performance liquid chromatography (HPLC) (Column: SB-806HQ (manufactured by Shodex Co.), column temperature: room temperature (25°C), eluting solution: aqueous sodium chloride solution having a concentration of 0.1 mol/L (liter), flow rate: 1.0 ml/min, detector: RI detector), and the weight average molecular weight (Mw) of each fucoidan was analyzed by a multi-angle light scattering detector (DAWN EOS (Registered Trademark No. 4377194): manufactured by Wyatt Technology Corp.).
The results are shown in Table 1.

[Table 1]

| Name of Sample | Weight average molecular weight ($\times 10^{-5}$ Mw) |
|---|---|
| Ethanol precipitate of high molecular-weight fucoidan | 18.45 |
| Hydrolyzed fucoidan (at 70°C for one hour) | 11.28 |
| Hydrolyzed fucoidan (at 70°C for 2 hours) | 8.19 |
| Hydrolyzed fucoidan (at 90°C for one hour) | 5.30 |

<Reference Example 1>

Using the high-performance liquid chromatography, the column, and the RI detector used in Example 3, the weight average molecular weight of the high-molecular fucoidan was relatively obtained using commercially available pullulan (molecular weight of 180 to 788,000) as a standard. As a result, it was 17,400,000.

<Example 4-1> Measurement of Viscosity

With respect to the aqueous high molecular-weight fucoidan solution (weight of dried residue: 1.2% by weight) prepared in Example 1, the respective aqueous hydrolyzed fucoidan solutions prepared in Example 2 and the aqueous acid-extracted fucoidan solution prepared in Comparative Example 1,viscosity was measured using a C-type viscometer at 20 rpm at 25°C.
The results are shown in Table 2.

[Table 2]

| Name of Sample | Viscosity (mPa·s) |
|---|---|
| Aqueous high molecular-weight fucoidan solution (Example 1) | 1,080 |
| Aqueous hydrolyzed fucoidan solution (at 70°C for one hour) | 464 |
| Aqueous hydrolyzed fucoidan solution (at 70°C for 2 hours) | 60 |
| Aqueous hydrolyzed fucoidan solution (at 90°C for one hour) | 2 |
| Aqueous acid-extracted fucoidan solution | 1 |

It was demonstrated that the aqueous high molecular-weight fucoidan solution prepared in Example 1 shows high viscosity, whereas, the aqueous hydrolyzed fucoidan solutions prepared in Comparative Example 2 and the aqueous acid-extracted fucoidan solution prepared in Comparative Example 1 show low viscosity.

<Example 4-2>

Ethanol was added to a raw alga body of Cladosiphon novae-caledoniae so as to obtain a concentration of 50% by weight. After dipping the raw mozuku seaweed for 30 minutes for discoloration, as much ethanol was removed as possible by centrifugal filtration to prepare processed mozuku seaweed.
150 g of the processed mozuku seaweed was put in 1, 350 ml of water and then 13.2 g of hydrogen peroxide water having a concentration of 30% by weight and 0.84 g of potassium hydroxide were added, followed by stirring at 90 to 95°C for one hour. After cooling to 50°C, the residue was removed by filtration.

EP 1 854 813 A1

Since the resulting filtrate was pH 6.4, 1 mol/L (liter) of an aqueous potassium hydroxide solution was used to adjusted the pH to 6.8.

Then, the filtrate was concentrated to about 1/5 of the original volume using an ultrafiltration membrane having a fractional molecular weight of 10,000.

The resulting aqueous solution was adjusted to pH 6.6 using 1 mol/L (liter) of dilute sulfuric acid.

Furthermore, the solution was treated with catalase to decompose peroxide so as to obtain an aqueous fucoidan solution. The weight of the dried residue of the aqueous fucoidan solution (at 70°C for 8 hours under normal pressure) was 1.88% by weight based on the original aqueous solution.

Moreover, fucoidan in the aqueous fucoidan solution was ethanol-precipitated in the same manner as Example 2 and measured for weight average molecular weight by a multi-angle light scattering detector. As a result, it was $13.0 \times 10^5$ (see, Example 3).

The viscosity of the aqueous fucoidan solution was measured in the same manner as Example 4-1. As a result, it was 1,070 mPa·s.

<Example 4-3>

Ethanol was added to a raw alga body Cladosiphon novae-caledoniae so as to obtain a concentration of 50% by weight. After dipping the raw mozuku seaweed for 30 minutes for discoloration, as much ethanol was removed as possible by centrifugal filtration to prepare processed mozuku seaweed.

150 g of the processed mozuku seaweed was put in 1, 350 ml of water and then 13.2 g of hydrogen peroxide water having a concentration of 30% by weight and 0.84 g of potassium hydroxide were added, followed by stirring at 90 to 95°C for one hour. After cooling to 50°C, the residue was removed by filtration.

Since the pH of the resulting filtrate was 6.4, 1 mol/L (liter) of an aqueous potassium hydroxide solution was used to adjust the pH to 7.5.

Then, the filtrate was concentrated to about 1/5 of the original volume using an ultrafiltration membrane having a fractional molecular weight of 10,000.

The resulting aqueous solution was adjusted to pH 6.7 using 1 mol/L (liter) of dilute sulfuric acid.

Furthermore, the solution was treated with catalase to decompose peroxide so as to obtain an aqueous fucoidan solution. The weight of the dried residue of the aqueous fucoidan solution (at 70°C for 8 hours under normal pressure) was 1.51% by weight based on the original aqueous solution.

Moreover, fucoidan in the aqueous fucoidan solution was ethanol-precipitated in the same manner as Example 2, and measured for weight average molecular weight by a multi-angle light scattering detector. As a result, it was $13.2 \times 10^5$ (see, Example 3).

The viscosity of the aqueous fucoidan solution was measured in the same manner as Example 4-1. As a result, it was 1,050 mPa·s.

<Example 4-4>

Ethanol was added to a raw alga body of Cladosiphon novae-caledoniae so as to obtain a concentration of 50% by weight. After dipping the raw mozuku seaweed for 30 minutes for discoloration, as much ethanol was removed as possible by centrifugal filtration to prepare processed mozuku seaweed. Then, each of the resulting 5 lots of processed mozuku seaweed was used to prepare an aqueous high-molecular weight fucoidan solution in the same manner as in Example 1. Moreover, the weight and the viscosity of the dried residue of each solution was examined. The weight of the dried residue is expressed by the weight ratio (% by weight) of the residue dried at 70°C for 8 hours under normal pressure based on the weight of the original aqueous solution, and the viscosity of each solution was measured at 25°C using a C-type viscometer.

[Table 3]

| Lot No. | Weight of dried residue (% by weight) | Viscosity (mPa·s) |
| --- | --- | --- |
| Lot 1 | 1.88 | 1,070 |
| Lot 2 | 1.51 | 1,050 |
| Lot 3 | 1.49 | 970 |
| Lot 4 | 1.00 | 660 |
| Lot 5 | 1.77 | 686 |

7

<Example 5> Preparation of Skin Lotion

(1) Preparation of Skin Lotion of High molecular-weight Fucoidan

0.05 g of phenoxy ethanol was added to 9.95 g of the aqueous high molecular-weight fucoidan solution (weight of dried residue: 1.2% by weight) prepared in Example 1 to prepare a skin lotion of high molecular-weight fucoidan. Hereinafter, the solution is referred to as a "skin lotion of high molecular-weight fucoidan".

(2) Preparation of Skin Lotion of Hydrolyzed Fucoidan

0.6 g of the ethanol precipitate of the hydrolyzed fucoidan (at 90°C for one hour) obtained in Comparative Example 2 was dissolved in purified water (weight of dried residue: 1.2% by weight) to make a total weight of 49.75 g, and then 0.25 g of phenoxy ethanol was added to prepare a skin lotion of hydrolyzed fucoidan.
Hereinafter, the resulting solution is referred to as a "skin lotion of hydrolyzed fucoidan".

<Example 6> Evaluation of Sensory Effect

One drop of each skin lotion prepared in Example 5 or purified water was applied to the back of the hands of seven panelists (36 to 59 years old) to evalutate the resulting tactile sensation, smoothness of the skin and moist feeling. The panelists' evaluations are based on the following criteria: agreeable tactile sensation (1), ordinary tactile sensation (0) disagreeable tactile sensation (-1), presence of smoothness and moist feeling (1) and absence of smoothness and moist feeling (0) and their answers were counted.
The results are shown in Table 4.
[Table 4]

| Name of sample | Tactile sensation | Smoothness | Moist feeling |
|---|---|---|---|
| Skin lotion of high molecular-weight fucoidan | 6 | 5 | 5 |
| Skin lotion of hydrolyzed fucoidan | 4 | 2 | 3 |
| Purified water | 0 | 0 | 0 |

It was confirmed that the skin lotion of high molecular-weight fucoidan exhibits good tactile sensation, smoothness and moist feeling as compared with the skin lotion of hydrolyzed fucoidan.

<Example 7> Evaluation of Moisture Retention

Immediate moisture retention of the aqueous high molecular-weight fucoidan solution prepared in Example 1 was evaluated by five panelists (three males and two females, 26 to 58 years of age). That is, in this evaluation, the aqueous high molecular-weight fucoidan solution or purified water was applied to the inside of the forearm in an amount of 10 mg/cm$^2$. The moisture content in the surface of the skin was measured 15 minutes after application, then 30 minutes, and 45 minutes, using an apparatus for measuring moisture content in the skin surface horny layer (SKICON 200: I.B.S. Co., Ltd.)
The results are shown in Table 5.
[Table 5]

| Name of sample | Measuring time | Moisture content |
|---|---|---|
| Purified water | Initial value | 100.0% |
| | After 15 minutes | 116.2% |
| | After 30 minutes | 107.4% |
| | After 45 minutes | 103.3% |
| Aqueous high | Initial value | 100.0% |

(continued)

| Name of sample | Measuring time | Moisture content |
|---|---|---|
| molecular-weight fucoidan solution | After 15 minutes | 164.5% |
| | After 30 minutes | 152.0% |
| | After 45 minutes | 147.2% |

It was shown that the aqueous high molecular-weight fucoidan solution exhibits excellent moisture retention as compared with purified water at all these points of time, or 15, 30 and 45 minutes after application.

<Example 8> Measurement of Hyaluronidase Inhibitory Activity

In accordance with the method described in Patent Document 13, inhibitory activity in the activation stage of inactive hyaluronidase by Compound 48/80 was measured usinghyaluronidase (Sigma, Type IV-S) derived frombovine testis. That is, in this example, the ethanol precipitate of the high molecular-weight fucoidan obtained in Example 2 and the ethanol precipitate (hydrolyzed fucoidan (at 90°C for one hour)) of hydrolyzed fucoidan (at 90°C for one hour) in Comparative Example 2 were each dissolved in 0.2 ml of an acetate buffer (PH4.0) having a concentration of 0.1 1 mol/L (liter). The resulting solutions were poured in test tubes and hyaluronidase (100 units) dissolved in 0.1 ml of the same buffer was added, followed by incubation at 37°C for 20 minutes.
Then, Compound 48/80 (0.1 mg) dissolved in 0.2 ml of the same buffer was added, followed by incubation at 37°C for 20 minutes.
A sodium hyaluronate salt (0.4 mg) dissolved in 0.5 ml of the same buffer was added to the solutions, followed by incubation at 37°C for 40 minutes. Then, 0.2 ml of an aqueous sodium hydrate solution having a concentration of 0.4 mol/L (liter) was added to the solutions which were ice-cooled. Afterwards, 0.2 ml of a boric acid solution (4.5 g of potassium tetraborate tetrahydrate was dissolved in water to make a total amount of 100 ml) (pH 9.1) was added to the solutions, followed by boiling for 3 minutes.
After ice cooling, 6 ml of p-dimethylaminobenzaldehyde was added, followed by incubation at 37°C for 20 minutes. Finally, the solutions were measured for absorbency at 585 nm.
The inhibitory rate of Compound 48/80 was calculated by the following equation. An inhibition concentration of 50% (IC50) was obtained from an inhibition curve of hyaluronidase that was made based on the inhibitory rate in each concentration.
(Equation 1)

$$\text{Inhibitory Rate} = (A-B)/A \times 100 \ (\%)$$

A: Absorbency of a control(acetate buffer was used instead of fucoidan)
B: Absorbency of fucoidan solution

The results of the measurement are shown in Table 6.
[Table 6]

| Name of sample | IC50 (mg/ml) |
|---|---|
| Ethanol precipitate of high molecular-weight fucoidan (Example 2) | 0.13 |
| Hydrolyzed fucoidan (at 90°C for one hour) (Comparative Example 2) | 0.21 |

As is apparent from these results, hyaluronidase inhibitory activity of the high molecular-weight fucoidan was 1.6 times stronger than that of the hydrolyzed fucoidan.

<Example 9> Test of Storage Stability (Accelerated Test)

The skin lotion of the high molecular-weight fucoidan prepared in Example 5 was stored at a temperature of 40°C under light of 2, 000 Lux for 4 weeks to be evaluated for stability. Also, the skin lotion was measured for viscosity at a cone of 3 degrees at a rotational speed of 20 rpm. In addition, a visual evaluation was conducted on the appearance of the skin lotion.
The results are shown in Table 7.

[Table 7]

|  | Viscosity (mPa·s) | Appearance |
|---|---|---|
| Initial value | 350 | Colorless and transparent |
| After 4 weeks | 400 | Colorless and transparent |

After 4 weeks of storage at 40°C, no decrease in viscosity and changes in appearance were recognized and it was demonstrated that the skin lotion had excellent storage stability.

<Example 10> Evaluation of Moisture Retention (2) (Test different from Example 7)

The inside of the forearms of the subjects was washed with soap. After drying and conditioning at 21°C and a relative humidity of 50%, four circles having a diameter of 2 cm were drawn per testing area (two testing areas/arm) on both arms. Then aqueous 1 wt% solutions were prepared by dissolving the respective ethanol precipitate of the aqueous high molecular-weight fucoidan solution (Example 1), the aqueous hydrolyzed fucoidan solution (at 70°C for one hour), the aqueous hydrolyzed fucoidan solution (at 70°C for 2 hours) and the aqueous hydrolyzed fucoidan solution (at 90°C for one hour) in purified water. 10 μL of each solution as well as purified water were applied inside the circles. After the treatment, the subjects were allowed to rest under the above-described environment for 200 minutes and their skin moisture content was measured using CORNEOMETER CM825 (CK electronic GmbH) and the average value of the four testing areas was obtained. Assuming the numerical value of the area to which purified water was applied to be 1, the relative skin moisture content of the subjects was calculated.
The results are shown in Table 8.

[Table 8]

| Name of sample | Relative skin moisture content |
|---|---|
| Purified water | 1.00 |
| High molecular-weight fucoidan | 1.32 |
| Hydrolyzed fucoidan (at 70°C for one hour) | 1.09 |
| Hydrolyzed fucoidan (at 70°C, for 2 hour) | 1.11 |
| Hydrolyzed fucoidan (at 90°C for one hour) | 0.99 |

The high molecular-weight fucoidan exhibited significantly high moisture retention as compared to purified water and hydrolyzed fucoidan.

<Example 11> Evaluation of Moisture Retention

Purified water, aqueous 0.5 wt% solutions prepared by dissolving, in purified water, each of the high molecular-weight fucoidan, the hydrated fucoidans (at 70°C for one hour, at 70°C for 2 hours, and at 90°C for one hour), and the ethanol precipitates of each of the aqueous hydrolyzed fucoidan solution (at 70°C for 2 hours) and the aqueous hydrolyzed fucoidan solution (at 90°C for one hour) were each treated with filter paper in an amount of 10 μL. The weight of the filter paper was measured every one minute using an electronic balance at a temperature of 21°C and a relative humidity of 50%, and the evaporation amount of moisture was obtained. The evaporation rate of moisture was calculated based on the results from the first 10 minutes of measurement and the relative evaporation rate was obtained assuming the evaporation rate of purified water to be 1. The results are shown in Table 9.

[Table 9]

| Name of sample | Relative moisture evaporation rate |
|---|---|
| Purified water | 1.00 |
| High molecular-weight fucoidan | 0.64 |
| Hydrolyzed fucoidan (at 70°C for one hour) | 0.65 |
| Hydrolyzed fucoidan (at 70°C, for 2 hour) | 0.70 |
| Hydrolyzed fucoidan (at 90°C for one hour) | 0.89 |

<Example 12> Preparation of Hair Treatment Solutions

Hair treatment solutions were prepared by using the aqueous high molecular-weight fucoidan solution (weight of dried residue: 1.2% by weight) prepared in Example 1, the aqueous hydrolyzed fucoidan solution (at 90°C for one hour) prepared in Example 2, purified water and ethanol.
The compositions of the hair treatment solutions are each shown in Table 10.
[Table 10]

| Hair treatment solutions | Aqueous fucoidan solution | Purified water | Ethanol |
|---|---|---|---|
| A | 20 | 60 | 20 |
| B | 20 | 60 | 20 |
| Note: The values in the table represent weight proportions of the aqueous fucoidan solution, purified water and ethanol. | | | |

In hair treatment solution A, the aqueous high molecular-weight fucoidan solution (Example 1) was used, and the aqueous hydrolyzed fucoidan solution (at 90°C for one hour) was used in hair treatment solution B.

<Example 13> Sensory Evaluation Using Damaged Human Hair

Damaged human hair was prepared by treating black human hair (10 g, manufactured by Beaulax) with a commercially available breach five times. After washing and drying, the hair was spray-treated with 2 ml of hair treatment solution A in Example 12 and dried. After drying, the hair treated with hair treatment solution A was compared to hair treated with hair treatment solution B in terms of smoothness to fingertips, moist feeling and flexibility. The results are shown in Table 11.
In this table, the solutions were rated in comparison with Comparative Example 2, with "Excellent" meaning superior, "Good" meaning of the same level, and "Poor" meaing inferior.
[Table 11]

| Name of sample | Smoothness | Moist feeling | Flexibility |
|---|---|---|---|
| Hair treatment solution A | Excellent | Excellent | Excellent |
| Hair treatment solution B | Good | Good | Excellent |

<Example 14> Evaluation of Cell Activation

Seeding of human skin fibroblasts (NB1RGB cell line) was performed on a 96-well plate at $5.0 \times 10^3$ cells/well, and the fibroblasts were precultured for 48 hours in DMEM medium (1% bovine serum). The medium was changed to DMEM medium containing a predetermined concentration of ethanol precipitates of each of the aqueous high molecular-weight fucoidan solution (weight of dried residue: 1.2% by weight) prepared in Example 1, and the aqueous hydrolyzed fucoidan solution (at 70°C for one hour) and the aqueous hydrolyzed fucoidan solution (at 70°C, for 2 hours), which were prepared in Example 2. Then, incubation was performed for 48 hours and the fibroblasts were subjected to WST-1 assay. Cell activating activity was expressed as a relative value assuming the value of a negative control (fucoidan additive-free DMEM medium (1% bovine serum)) to be 100%. The assay was performed twice continuously on each of the samples. The results are shown in Table 12.

[Table 12]

| Name of sample | Concentration (μg/ml) | | | Positive control |
|---|---|---|---|---|
| | 2 | 20 | 200 | |
| High molecular-weight fucoidan | 123.9 | 121.7 | 111.9 | 161.5 |
| Hydrolyzed fucoidan (at 70°C for one hour) | 101.1 | 88.8 | 38.7 | 139.6 |
| Hydrolyzed fucoidan (at 70°C for two hours) | 105.1 | 96.4 | 96.4 | 139.5 |

The values in the table represent relative values assuming the value of a positive control (DMEM medium (1% bovine serum)) to be 100%.

<Example 15> Constituent Component of Fucoidan

The high molecular-weight fucoidan precipitate obtained in Example 2 was hydrolyzed using formic acid (at 135°C for 6 hours), followed by hydrolysis using 2M trifluoroacetic acid (at 120°C for 6 hours), which was then analyzed by HPLC (Column: Cosmosil Sugar-D, manufactured by Nakalai Tesque Co., Ltd., Solvent: acetonitrile: 10mM phosphate buffer = 75:25, flow rate: 1.0 mL/min, RI detector). As a result, it was found that fucose was the main constituent saccharide of fucoidan which contained glucuronic acid also. Moreover, the high molecular-weight fucoidan precipitate was analyzed by the rhodizonic acid method, and measured as follows for the sulfate group content. That is, the measurement was performed at 60°C by NMR (500 MHz, manufactured by Japan Electron Optics Laboratory Co., Ltd.) by dissolving the precipitate in Denterium Oxide. Then, the acetyl group content was determined from the area ratio of the methyl group peak of fucose at about 1.3 ppm to the acetyl group peak at 2.2 ppm. The content ratio of glucuronic acid to acetyl group in the high molecular-weight fucoidan precipitate was determined assuming that the amount of fucose to be 1.0. The results are shown in Table 13.

[Table 13]

| Constituent molecular ratio | | |
|---|---|---|
| Fucose | Glucuronic acid | Acetyl group |
| 1.0 | 0.11 | 0.10 |

It was found that the sulfate group content was 16.9% by weight based on the precipitate.

**Claims**

1. A method for producing fucoidan comprising the steps of:

   performing hot water extraction of a mozuku alga body of the genus Cladosiphon at pH 6.0 or higher, removing low-molecular weight compounds while maintaining the hot water at pH 6.5 or higher, and adjusting the hot water to a final pH of 6.5 or higher to obtain fucoidan having a weight average molecular weight of 1,000,000 to 2,000,000.

2. Fucoidan having a weight average molecular weight of 1,000,000 to 2,000,000 obtained by a method for producing fucoidan comprising the steps of:

   performing hot water extraction of a mozuku alga body of the genus Cladosiphon at pH 6.0 or higher, removing low-molecular weight compounds while maintaining the hot water at pH 6.5 or higher, and adjusting the hot water to a final pH of 6.5 or higher.

3. Fucoidan having a weight average molecular weight of 1,000,000 to 2,000,000.

4. Fucoidan according to claim 2 or 3, wherein an aqueous solution thereof having a dried residue weighing 1.0 to 2.0% by weight has a viscosity of from 600 to 1,500 mPa·s as measured at 20 rpm and 25°C using a C-type viscometer.

5. A fucoidan-containing composition, comprising fucoidan according to any one of claims 2 to 4.

6. The fucoidan-containing composition according to claim 5, wherein the fucoidan-containing composition is a cosmetic composition, a skin cell activator or an anti-allergic agent.

**EP 1 854 813 A1**

| **INTERNATIONAL SEARCH REPORT** | International application No. |
|---|---|
| | PCT/JP2006/303834 |

**A. CLASSIFICATION OF SUBJECT MATTER**
*C08B37/00*(2006.01), *A61K31/737*(2006.01), *A61K8/73*(2006.01), *A61P37/08*
(2006.01), *A61P43/00*(2006.01), *A61Q5/00*(2006.01), *A61Q19/00*(2006.01)

According to International Patent Classification (IPC) or to both national classification and IPC

**B. FIELDS SEARCHED**

Minimum documentation searched (classification system followed by classification symbols)
A61K31/737, A61K8/73, C08B37/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
WPI

**C. DOCUMENTS CONSIDERED TO BE RELEVANT**

| Category* | Citation of document, with indication, where appropriate, of the relevant passages | Relevant to claim No. |
|---|---|---|
| X | JP 2003-300887 A (Tanguruuddo Kabushiki Kaisha), 21 October, 2003 (21.10.03), Full text (Family: none) | 1-6 |
| A | JP 2004-75595 A (Kabushiki Kaisha Kaisanbutsu no Kimuraya), 11 March, 2004 (11.03.04), Par. No. [0029] (Family: none) | 1-6 |
| A | JP 2002-171943 A (Kabushiki Kaisha Supesu Shoji), 18 June, 2002 (18.06.02), Full text (Family: none) | 1-6 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 May, 2006 (10.05.06) | 23 May, 2006 (23.05.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10191940 A
- JP 11035591 A
- JP 2000239301 A
- JP 2000239302 A
- JP 2000351801 A
- JP H10195106 A
- JP H10237103 A
- JP H07138166 A
- JP H1070970 A
- JP H0611763 B
- JP H0714850 B
- JP H1121247 A
- JP 2001151788 A

**Non-patent literature cited in the description**

- New Cosmetology. Nanzando, 1999, 5-7147-148
- *Riken Mekabufukoidan,* 22 February 2006, ht-tp://www.rikenvitamin.jp/prouse/health/meka bufu-koidan.html